# EUROPEAN PATENT SPECIFICATION

(11) **EP 2 552 371 B1**
(45) Date of publication and mention of the grant of the patent: **24.06.2020**
(21) Application number: 10713525.3
(22) Date of filing: 31.03.2010
(51) Int. Cl.: A61F 13/00

(54) **A WOUND CARE DRESSING, A METHOD AND A PRODUCTION LINE FOR MANUFACTURING THE WOUND CARE DRESSING**
WUNDVERBAND, VERFAHREN UND PRODUKTIONSANLAGE ZUR HERSTELLUNG DES WUNDVERBANDES
PENSEMENT POUR PLAIE, PROCÉDÉ ET LIGNE DE PRODUCTION DESTINÉS À LA FABRICATION LE PENSEMENT POUR PLAIE

(43) Date of publication of application: 06.02.2013
(73) Proprietor: Pharmaplast SAE, Alexandria 23512 (EG)
(72) Inventor: ATTEIA, Mamdouh H., Alexandria 23512 (EG)
(74) Representative: Holme Patent A/S
(86) International application number: PCT/IB2010/051414
(87) International publication number: WO 2011/121394

(56) References cited:
- WO-A2-2007/113597
- US-A- 4 212 296
- US-A- 5 974 344
- US-A1- 2005 074 482

## Description

The present invention relates to a wound care dressing comprising at least- a separating film layer sheet having a topside and a bottom side both provided with an adhesive, a hydrogel layer sheet attached to the bottom side of the separating film layer sheet to form a combined matrix layer, and where- the matrix layer has a plurality of perforations, wherein a polyurethane foam layer sheet is attached to the topside of the separating layer sheet and covered by a polyurethane cover film layer sheet, which allows passage of water vapor.

Within the scope of the present invention the term "hydrogel" covers a highly hydrated cross-linked polymer gel in which the polymer chain holds many times its weight in trapped water. The hydrogels are obtained by combining a disperse phase of one or more polymers with a continuous water phase to obtain a colloid. Hydrogels are known to be useful for wound treatment due to a.o. absorption, desloughing and debriding capacities of necrotics and fibrotic tissue, as well as being effective in creating and maintaining a moist environment. Hydrogel dressings further have a pleasant soothing effect. Hydrogel dressings are non-adherent to the wound bed, maintain its integrity, so no residues are left on removal of the dressing, and can be removed without trauma or pain. Because hydrogels already contain up to 95% water, hydrogels cannot absorb much exudate. Thus hydrogel dressings are commonly used for dry or minimal drainage wounds only, e.g. pressure ulcers, skin tears, surgical wounds, and burns.

Due to these properties hydrogel dressings have become important in wound care regimens.

Attempts have been made to combine such hydrogels with absorbent means in absorbent wound dressings to also obtain control of absorbing quality and property, however wound dressings including both hydrogel and absorbent means have the disadvantage that the hydrogel dries out due to absorption of water trapped in the hydrogel matrix by the absorbent means.

A system that provides for breathing through a patch means adhesively attached to a part of the skin is known from US patent document US 5,974,344 A. This known system includes a wound care electrode including a flexible, electrically conductive body in a layered structure. The conductive body is inserted between an electrically conductive hydrogel layer and a flexible, nonconductive, separating polyethylene layer. A flexible, absorbent hydrocolloid dressing is secured to the top of the separating layer, said absorbent hydrocollid dressing is composed of a 1.5 mm absorbent hydrocolloid jelly layer applied in liquid stage to 0.5 mm polyurethane foam and provided with a polyethylene layer on the opposite side. The electrically conductive body, the electrically conductive gel layer, and the separating layer have axially aligned perforations for conveying seepage from the skin of a user to the absorbent hydrocolloid dressing and the polyurethane foam. During manufacturing the open-celled nature of the polyurethane foam permits the unset hydrocolloid to partially flow into the polyurethane foam and strongly bind thereto. While the hydrocolloid is still partial liquid, before setting, the polyethylene separating layer is adhered to the absorbent hydrocolloid dressing layer. In the final wound care electrode the absorbent capacity of the polyurethane foam is substantially reduced due to the fact that the polyurethane foam absorbs a considerable amount of the liquid phase of the hydrocolloid jelly matrix. Moreover, when unset hydrocolloid meets the perforated polyethylene layer, the unset hydrocolloid will clot the perforations, and negatively affect the moisture seepage paths towards the polyurethane foam as well as the absorbing capacity in total.

Hydrocolloid dressings have many advantages, however in contrast to hydrogel dressings, hydrocolloid dressings are occlusive and do not allow the skin to breath. Hydrocolloids can cause the pH of the wound surface to drop. The resulting acidic environment contributes to inhibit bacteria growth. Due to being occlusive, hydrocolloids cannot be used if the wound or surrounding skin is infected or on necrotic tissue. WO 2007/113597 A2 discloses a wound care dressing comprising a film layer sheet, a silicone gel layer sheet, and a foam layer sheet covered by a cover film layer sheet.

Within the field of wound care dressings there is a need for improvements, in particular for wound care dressings for exuding wounds.

In a first aspect according to the present invention is provided a wound care dressing of the kind mentioned in the opening paragrah, wound care dressing has ccontinuous long term improved high absorption capacity and allows water vapour to escape.

In a second aspect according to the present invention is provided a wound care dressing of the kind mentioned in the opening paragrah that can be used for exuding wounds.

In a third aspect according to the present invention is provided a wound care dressing of the kind mentioned in the opening paragrah that, without increased risk of infection, can remain on a wound for longer time before being replaced than known wound care dressings.

In a fourth aspect according to the present invention is provided a wound care dressing of the kind mentioned in the opening paragrah that promotes granulation and reepithelisation at a wound site.

In a fifth aspect according to the present invention is provided a wound care dressing of the kind mentioned in the opening paragrah that promotes autolytic debridement in wounds with necrotic tissue.

In a sixth aspect according to the present invention is provided a wound care dressing of the kind mentioned in the opening paragrah that prevents micro-organisms and water entry into a wound, but allow water vapour to pass.

In a seventh aspect according to the present invention is provided a wound care dressing of the kind mentioned in the opening paragrah having improved patient comfort, is easy to apply and painless to remove.

The novel and unique whereby these and further aspects are achieved according to the present invention consist in the fact that a foam layer sheet is attached to the topside of the separating layer sheet and covered by a cover film layer sheet.

The aligned perforations through the laminated hydrogel layer sheet and separating layer sheet of the inventive wound care dressing allows fluid passage from the wound towards the foam layer sheet, allowing exudates and accumulated moisture to be absorbed instantly by the foam layer sheet, thus risk of maceration or hindering the granulation process is avoided.

The separating film layer sheet is provided with adhesive on both sides in order to be adhered intimately to both the hydrogel layer sheet and the foam layer sheet instead of being laminated by means of hydrogel in liquid or unset form, as in the prior art. The perforations, that extend through the matrix layer sheet are, at least prior to use, substantially clear of undesired hydrogel entrance or penetration, enabling the perforations to act as effective rapid-acting capillary paths, which allows the foam to imbibe fluid from the wound bed. Because the foam layer sheet is kept away of direct contact with the hydrogel layer sheet the aqueous phase of the hydrogel does not impair all or part of the foam's absorption capacity during manufacturing and storage. During use fluid, such as moisture and exudate from the wound, is allowed to pass through the perforations into the foam layer while the hydrogel maintains substantially all its beneficial skin and debris properties mentioned above. The topmost cover film layer sheet constitutes an expedient barrier to the exterior environment and prevents the outwards facing foam layer sheet from absorbing moisture and humid from said exterior environment, thus it prevents the wound from moisture and bacterial invasion. The capillary action of the perforations contributes to conveying fluid from the wound towards the foam layer sheet and further towards the cover film layer sheet that protects the wound dressing from above.

The foam layer sheet is a polyurethane foam layer sheet. Polyurethane foam is a spongy cellular material used for many kinds of padding due to its hydrophilic properties. Within the scope of the present invention other kinds of foam, compounds, materials or items having similar properties can also be implemented in the wound care dressing. Thus the term "foam" should be construed in its broadest sense. Foam materials can be selected to be flexible, compressible, non-compressible and have more or less memory shape properties that allows the foam to restore its original shape after compression, e.g. in case of accidental hit or if the user slept on the wound care dressing.

Amount and rate of fluid at a wound bed vary, which conventionally necessitates frequent changes of wound care dressings, as well as a range of different types of dressings to treat different wounds. The thickness of the layer sheets of the wound care dressing according to the present invention can be selected to maintain a desired moist level at the wound bed and around the wound to meet such demands.

The cover film layer sheet is a polyurethane film layer sheet that allows passage of water vapor, and/or is bacteria proof and/or is water proof, however other cover film layer sheets that allows passage of water vapour is also contemplated within the scope of the present invention.

An appropriate thickness of the foam layer sheet may be between 2 - 7 mm, preferably between 3 - 6 mm and most preferred between 4 - 5 mm in order that the absorbing property is favourable for slight to moderate exuding wounds, however wound dressings can be made with different thicknesses of foam layer sheets to be used on more or less moist or exuding wounds. For example a thickness of 2 mm suits a very moderate moist wound, a thickness of 4 mm is appropriate for medium exuding wounds, and even larger thicknesses can be incorporated for the purpose of heavily exuding or moist wounds.

The thickness of the cover film layer sheet covering the foam layer sheet can be between 15 - 35 µm, preferably between 20 - 30 µm and most preferred between 22 - 28 µm. A thickness of for example about 25 µm is a frequent choice because this medium thickness is easy to handle and obtainable as stock goods.

The cover film layer sheet advantageously can be selected to serve as a barrier to passage of microorganisms from the environment, while at the same time controlling degree of water evaporation, to preserve a continuous high and/or adjusted absorbing capacity of the foam layer sheet. The wound care dressing according to the present invention is thus designed to stay on a wound for longer time than known wound care dressings without increased risk of aggravating infection conditions. Various wound care dressing can be composed. Some can be designed to be able to control escape of wound fluid from the wound bed, e.g. the cover film layer sheet may block excessive escape of fluid if a moist environment is aimed for, and other be designed to promote escape of fluid if a dry environment is aimed for to speed up granulation.

Preferred materials for the separating film layer sheet can be a polyethylene film layer sheet or a polyurethane layer sheet, however other plastic materials having same physical and chemical properties are also contemplated within the scope of the present invention.

A suitable thickness of the separating film layer sheet including the adhesive is between 40 - 100 µm, preferably between 50 - 80 µm, and most preferred between 60 - 70 µm. These intervals of thickness are suitable for preventing the aqueous phase of the hydrogel layer sheet from being accessed and absorbed by the foam.

The thickness of the hydrogel layer sheet may be 1 - 5 mm, preferably 2 - 4 mm and most preferred between 2.5 - 3.5 mm depending on choice and intended purpose of the wound care dressing.

The cover layer film sheet, which is a polyurethane film layer sheet, may advantageously have a Moisture Vapour Transmission Rate (MVTR) of at least 3000g/m²/24 hour to allow evaporation, thereby maintaining prolonged absorption capacity of the foam layer sheet. Thus the preferred material of the cover layer film sheet is able to maintain an evaporation degree that restores and maintains at least some of the absorption capacity of the foam layer sheet, and preferably most of the absorption capacity of the foam layer sheet for a sustained period of time, e.g. up to one week.

The foam of the foam layer sheet may have a MVTR of at least 3500g/m²/24 hour. Preferably the foam layer sheet is a polyurethane foam layer sheet, however other open-celled materials having the same absorption capacity is contemplated within the scope of the present invention. The foam layer sheet can also be a laminated structure composed of several absorbing layers having the same or different porosity.

Due to at least the combined properties of evaporation through the cover layer sheet, the capillary action of the perforations, and the absorption properties of the foam layer sheet the wound care dressing need not be replaced as frequently as prior art wound dressings. The patient also enjoys the benefits of the hydrogel in debridement as the hyrogel is active in removal of necrotic tissue, exudate, and metabolic waste from a wound, as well as the hyrogel provides optimum temperatures for wound healing. The wound care dressing according to the present invention provides optimal wound management.

The hydrogel layer sheet of the wound care dressing may be chosen to be non-tacky to allow removal without trauma or pain induction. Furthermore, the hydrogel matrix is designed to keep its integrity after absorption of exudates. Accordingly, no hydrogel residues are left on the wound bed upon removal of the dressing, unlike wound dressings which cannot be removed from the wound as one piece, leaving residues that can be removed only by irrigation which is a painful and lengthy process that impairs both the patient and the nursery quality of life.

Preferably, such hydrogel may be composed of urethane prepolymer having unreacted isocyanate groups, preferably diisocyanate groups.

The perforations through the matrix layer may in an expedient and inexpensive embodiment be made by ultrasonic perforation, because perforation made by ultrasonic emitter does not damage the hydrogel or hinder the hydrogel integrity, as e.g. many mechanical punching means do.

If the perforations are substantially equally distributed over the area of the matrix layer the wound care dressing can imbibe wound exudates equally over the entire area of the matrix layer.

Preferred perforations may have a diameter or transverse section of substantially between 2 - 4 mm, preferably between 2.5 - 3.5 mm, and most preferred 3 mm, to allow exudate or moist to pass on to the foam layer sheet at a sustained, steady and beneficial rate that is enough to keep moisture content at the wound bed at the desired level to prevent desiccation of the wound.

In an exemplary embodiment the perforations can be provided at a mutual distance of about 8 - 10 mm. In this embodiment, or in an alternative embodiment depending on the diameter or traverse sections of the perforations, the number of perforations can be about 80 - 120/100 cm² matrix layer. Within the scope of the present invention the perforations may have the same or different diameter or traverse sections and be provided in predefined patterns. For example the density of perforations may be larger at selected areas, e.g. at the centre area of the matrix layer where the need for capillary action and imbibe may be the highest. Thus, any combination of size of perforations, density of perforations and patterns of perforations are foreseen within the scope of the present invention.

In order to be able to store multiple wound care dressings and otherwise ensure that the wound care dressing does not stick unintentionally to undesired surfaces, the hydrogel layer sheet can be covered with a first release liner.

A release liner, that is especially easy to use when applying the wound care dressing on the wound bed, may comprise a first flap and a second flap, optionally the first flap and the second flap overlaps at least partly.

In an island pad embodiment the cover layer film sheet may expediently extend with a border beyond the perimeter of the combined matrix layer and foam layer sheet to provide an additional skin attachment border film. The adhesive border is furthermore very easy to peel off the skin for removal or replacement of the wound care dressing.

A method of manufacturing the wound care dressing described above comprises the steps of
(a) providing a width of a matrix layer protected by first low release liners on both sides,
(b) providing a plurality of perforations through the matrix layer,
(c) removing the first low release liner from the separating film layer side of the matrix layer,
(d) providing either
   - a width of foam layer, or
   - a width of a laminate comprising a foam layer combined with a cover film layer, and a backing layer on the cover film layer side of the laminate,
(e) combining the width of perforated matrix layer with either
   - the width of foam layer, or
   - the width of laminate
   to produce a width of wound care dressing material,
(f) optionally removing the backing layer from the cover film layer, and either maintaining the first low release liner on the hydrogel side of the matrix layer or substituting the first low release liner with a second low release liner comprising two flaps, and
(g) obtaining wound care dressings by end cutting the width of wound care dressing material including a cover film layer.

In this manner the wound care dressing according to the present invention can be manufactured in a continuous process using feedstock materials in which the layers of the final wound care dressing are protected initially by low release liners, so that no layers unintentionally stick to other objects than intended for creating the wound care dressings while the first release liner is simple to remove when the protected layers are to be laminated with other layers. Even layers as thin as discussed above can be laminated to each other, and the perforations can be made as an integrated step in the method at high speed.

An exemplary adhesive is e.g. the hypoallergenic adhesive Code: WD2184 obtainable from Everfront Industrial Co., Ltd., P.O. Box 43-435, Taipei, which also is the provider of a preferred clear polyethylen film layer, a Poly (Ethylene Vinyl Acetate) of 60 µm.

Within the scope of the present invention the term "acrylic-based adhesive" is to be understood as an adhesive having a content of an acrylic polymer. Acrylic-based adhesives have the advantage of setting rapidly, being very strong due to heavy cross-linking, have high peel and impact strength, tolerates high temperatures and thus have good environmental resistance. Acrylic-based adhesives are the preferred adhesive on the separating layer, such as a polyethylene film layer or a polyurethane film layer.

In the preferred embodiment the foam layer is the polyurethane foam layer and the cover film layer is the polyurethane film layer having the properties described above for the wound care dressing. Preferred polyurethane film layers includes but are not limited to bacterial and waterproof polyurethane film layers.

A preferred polyurethane foam layer is obtainable from Filtrona Richmond Inc. as product no. MS 50P or from Rynel, 11 Twin Rivers Drive, Wiscasset, ME 04578, USA, as product no. 562-B. A preferred polyurethane film is obtainable from Cytec Industries, Anderlecht Str., 33 B-1620 Drogenbos, Belgium, as product no. Ucecoat AB5454.

In an advantageous embodiment the width of wound dressing material produced in step (e) can be wound up on an end bobbin prior to entering step (f), so that e.g. an island pad embodiment can be made in a two-stage procedure at distant locations as semifinished product and final wound care dressing, respectively.

In an embodiment where the backing layer is not removed from the cover film layer in step (f) island pads are provided from the end bobbin and laminated to the cover film, before the second release liners is applied on the skin-facing hydrogel side. The adhesive border extends beyond the perimeter of the island pad.

In order to ensure that the wound care dressing layers maintain their integrity during lamination and to sufficiently protect the hydrogel of the final wound care dressing, any of the first and/or second low release liners can be low release siliconized polyethylene or siliconized polyester film. Known release agents for release liners are crosslinkable silicone, however other coatings and materials that have a low surface energy can also be used in the method and the wound care dressing according to the present invention. A typical release liner is thus a polyethylene backing, but a plastic based carrier web material, which is coated with a release agent can also be used.

Particular trimmed perforations can be made through the matrix layer using ultrasound. Ultrasonic perforations have the advantage that the perforation is clean i.e. there are no perforation wastes resulting from punching. Moreover, traditional punching tools may partially perforate the matrix layer, leaving perforation wastes attached to the matrix that can only be removed by means of vacuum suction. Thus perforation by ultrasonic emitters is considered much easier and more accurate, especially when the matrix layer includes a hydrogel sheet, which may be damaged if squeezed under a traditional punching tool.

The number of perforations can for example be between about 80 - 120/ 100 cm² matrix layer for most wound dressings.

Maintenance of the integrity of the polyurethane film layer is important for the optimum performance of the final wound care dressing. In order that the polyurethane film layer not creases during carrying out the method the backing layer on the polyurethane film layer side of the polyurethane laminate is chosen as a paper backing, because such a paper backing is inexpensive, reliable and provides a suitable stiffness while still being able to be wound on rolls. When backed by a paper backing the polyurethane film layer has sufficient strength to withstand the manufacturing conditions when being supplied to the method and the production line at sustained high rate without the polyurethane layer being damaged.

To satisfy a common requirement for many medical purposes the wound care dressings obtained in step (g) can also be sterilised.

A production line for manufacturing the wound care dressing is also described.

The production line comprises at least
- a first station for unwinding a width of a matrix layer protected by first low release liners on both sides,
- a second station being a perforation unit for providing a plurality of perforations through the matrix layer,
- a third station for removing the first low release liner from the separating film layer side of the matrix layer,
- a fourth station for providing either
   - a width of a polyurethane foam layer, or
   - a width of a polyurethane laminate (27) comprising a polyurethane foam layer (14a) combined with a polyurethane film layer and a backing layer (31) on the polyurethane film layer side of said polyurethane laminate
- a fifth station for combining the width of perforated matrix layer with either
   - the width of polyurethane foam layer, or
   - the width of polyurethane laminate
   to produce a width of wound care dressing material,
- a sixth station for optionally removing the backing layer from the polyurethane film layer, and either maintaining the first low release liner on the hydrogel side of the matrix layer or substituting said first low release liner with a second release liner comprising two flaps, and
- a seventh station being and end cutting station for cutting out the wound care dressing of the wound care dressing material leaving the sixth station.

When producing the island pad embodiment the backing layer maintains on the width of polyurethane layer to provide structural integrity during transport into and through the production line.

By using a production line having a plurality of co-operating stations a high production speed can be maintained, and the wound care dressing can be made of e.g. commercially available laminates and materials. Polyurethane films having high moisture vapor transmission rate (MVTR) may be used, but such breathable polyurethane films are normally very thin which puts demand on the manufacturing process, because such films need to be very carefully treated. By supplying the layers for the final wound care dressing as widths of feedstock material protected and/or carried on release liners and/or backing layers that are removed/applied at the relevant production line station just prior or subsequent to the respective layers of the wound care dressing are subjected to further processing in said production line, the respective layers can be protected for the longest possible time during the method steps and through the production line, until they are laminated with other layers and second release liners, before being end cut to final shape.

Advantageously the end cut wound care dressing may proceed directly to an eighth station being a sterilization station.

In an embodiment the perforation unit may provide the plurality of perforations by means of ultrasound applied to the matrix layer laminate including first release liners on both sides, thereby allowing the production line to run at a continuously high speed expediently eliminating the need for the object to be perforated being stagnant as may be required if a mechanical punching means were used for creating the perforations. A further advantage is that no remains, such as lubricant or cleansing agent, are transferred from a corresponding mechanical means, for example punching pins, to the matrix layer during the unavoidable physical contact. By operating an ultrasonic source above the selected location clean perforations can be made rapidly without fracturing the matrix layer, which is protected on both sides by the first release liners. Thus a better perforation quality at a higher production speed than can be seen in the prior art can be obtained using ultrasound perforation.

The production line may include various auxiliary means for feeding, conveying, and transporting widths of feedstock material and intermediate laminates and webs through the production line.

Such auxiliary means may expediently include any of the following: that the first station comprises a first roller means for supplying the matrix layer, the third station comprises a first delaminating roll for winding a first release liner off the separating layer of the matrix layer, the fourth station comprises a second roller means for supplying the polyurethane laminate or the polyurethane foam, the fifth station has lamination means for laminating the matrix layer to the polyurethane laminate, the sixth station comprises a second delamination roll for winding the backing layer off the polyurethane laminate, the sixth station further comprises a third delamination roll for winding the first low release liner off the hydrogel layer of the matrix layer, and a third roller means for supplying a width of a second release liner in the form of a first flap and a second flap to said hydrogel layer.

In particular in a production line for producing the island pad embodiment, an end bobbin can be inserted between the fifth and sixth station to wind up wound dressing material for subsequent use in a further lamination process. The wound dressing material may be stored at the end bobbin for a period of time but preferably just briefly so that the hydrogel does not dry out. Temporary storage of the wound dressing material provides the further possibility of also being able to produce the final wound care dressing using two separate substations, one for making an initial trilaminate including the separating layer, the foam layer and the hydrogel layer, and another substation where the island pad is cut out by means of a die pad cutter, and a final laminate also including the polyurethane film layer can be made, as well as release liners also can be applied.

Advantageously the sixth station can further comprise rollers for winding up waste from any of the release liners subsequent to cutting.

The production line may thus also comprise conveyor means for transporting feedstock layers of matrix layer, polyurethane laminate, polyurethane foam and second low release liner, respectively, and intermediate products and webs through the production line, said conveyor means may advantageously comprises nip roller units.

In order to obtain the suitable size and shape of the final wound care dressing the width of wound care dressing material may be pad cut and/or end cut by die cutting said width of wound care dressing material into various sizes and/or shapes at the sixth and/or seventh station, respectively. The island pad and the final wound care dressing may thus be cut into any desirable shape including circular, rectangular, oval, sacral and concave or any other arbitrary shape.

An exemplary quadratic wound care dressing may be manufactured in a product range including dressings being 100 x 100 mm, 150 x 150 mm and 200 x 200 mm. An exemplary rectangular wound care dressing can be 100 x 200 mm or 200 x 300 mm. Thus, wound care dressings having very large sizes and high MVTR can be made according to the present invention. The breadth of the peripheral adhesive film border in the island pad embodiment may vary depending on the size of the wound care dressing. An exemplary breadth may be e.g. 10 to 50 mm for most of the above sizes of wound dressings.

The invention will now be described by way of exemplary embodiments with reference to the accompanying drawing, in which
fig. 1 shows, seen in perspective, an exploded view of a first embodiment of a wound care dressing according to the present invention,
fig. 2 shows, seen in perspective oblique from the side facing opposite the skin in use, the wound care dressing seen in fig. 1 in assembled state,
fig. 3 shows schematically a first production line for manufacturing the first embodiment of a wound care dressing according to the present invention,
fig. 4 shows, seen in perspective, an exploded view of a second embodiment of a wound care dressing according to the present invention,
fig. 5 shows, seen in perspective oblique from the side facing opposite the skin in use, the wound care dressing seen in fig. 4 in assembled state, and
fig. 6 and 7 shows in combination, schematically a second production line for manufacturing the second embodiment of a wound care dressing according to the present invention.

The wound care dressings according to the present invention are described in more detail below by way of exemplary designs. Although the wound care dressings are shown in the figures to be rectangular with smooth curves, the figures should not be construed to limit the design and outline of the wound care dressings according to the invention.

The thicknesses of the various layers are indicated by way of example. Also by way of example the foam layer sheet is described as a porous polyurethane foam layer sheet, the cover film sheet is described as a thin breathable polyurethane film layer sheet having a MVTR of 3000g/m²/24 hour and the exemplary low release liners are polyethylene siliconized release liners. However other foams, films and release liners having similar properties with respect to absorption, MVTR and releaseability is intended within the scope of the present invention. The examples given should not be construed to limit said scope. The exemplary separating layer is a polyethylene layer.

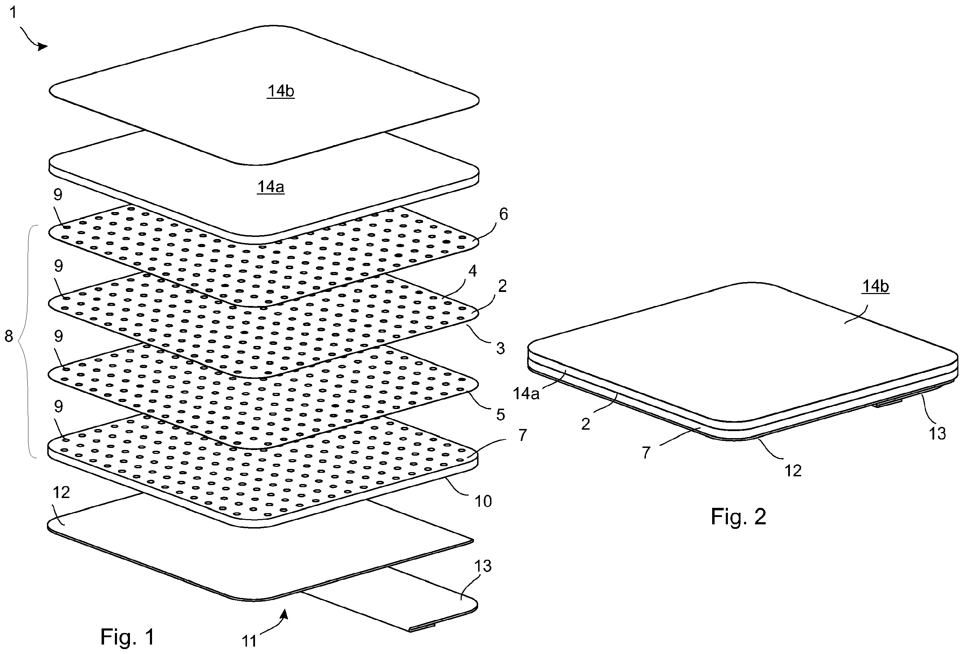

Fig. 1 shows an exploded view of an exemplary wound care dressing 1 consisting of a polyethylene film layer sheet 2, having a bottom skin-facing side 3 and an opposite topside 4 facing away from the wearer subsequent to application on a wound. The bottom side 3 is provided with a first adhesive 5 and the topside 4 is provided with a second adhesive 6, which may be the same as the first adhesive 5 or different. The combined exemplary thickness of the adhesive-coated 5,6, polyethylene layer 2 is 100 µm. The first adhesive 5 serves for gluing and attaching a hydrogel layer sheet 7 of e.g. about 3 mm to the polyethylene layer sheet 2 to achieve a matrix layer 8. The matrix layer 8 has a plurality of through-going holes or perforations 9, e.g. 3 mm in diameter, distributed evenly over the entire area of the matrix layer 8. Other distributions and patterns are foreseen within the scope of the present invention.

The skin-facing side 10 of the hydrogel layer sheet 7 is protected by a second low release silicon liner 11 consisting of a major first release liner flap 12 and a minor second release liner flap 13, the latter being intended for being initially grasped in relation to application of the wound care dressing 1 on a wound. The flaps 12,13 partly overlaps.

The topside 4 opposite the skin-facing bottom side 3 of the polyethylene layer sheet 2 is laminated to a polyurethane foam layer sheet 14a. Said polyurethane foam layer sheet 14a is mounted on top of the polyethylene layer sheet 2 by means of the adhesive 6. The polyurethane foam layer sheet 14a, which e.g. is 5 mm thick, is covered with a polyurethane film layer sheet 14b, e.g a polyurethane film layer sheet being about 25µm thick.

The wound care dressing 1 is seen in assembled state in fig. 2 in a perspective view. The combined thickness of the final wound care dressing 1 is between 8 - 10 mm including the release liners 12,13, however modifications within the claimed intervals of layer thicknesses will result in alternative final end product thicknesses.

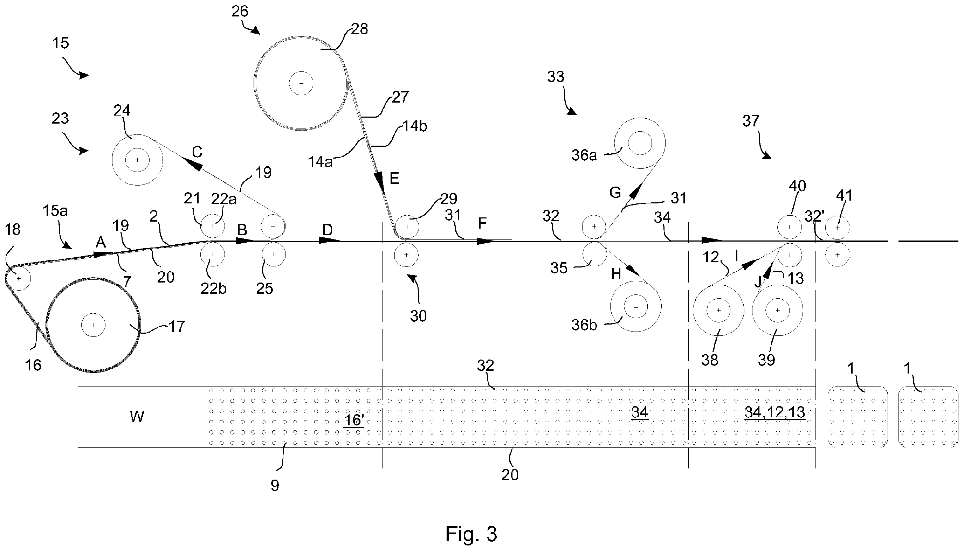

Fig. 3 shows schematically a production line 15 for manufacturing the first embodiment 1 of a wound care dressing.

The production line 15 has a first station 15a for, as indicated by arrow A, unwinding a width W of matrix layer 16 from the supply roll 17 via the conveyor roll 18, said supply roll 17 and conveyor roll 18 constituting the first roller means 17,18. The width W of matrix layer 16, which in the present example is a laminate composed of the layer for the polyethylene layer sheet 2 coated with adhesive 5,6 on opposing sides 3,4 and being united by means of said adhesive 5 on the skin-facing bottom side 3 to a width of hydrogel layer 7. The exterior side 10 of the hydrogel layer 7 and the adhesive 6 on the topside 4 of polyethylene layer sheet 2 are protected by first low release silicon liners 19,20, respectively, during storage and initial manipulation in the production line 15.

The width W of matrix layer 16 covered on both sides with respective first low release silicon liners 19,20 is conveyed to a second station 21, a perforation unit 21, preferably an ultrasound perforation unit, schematically indicated by the rollers 22a,22b.

After the perforations 9 have been made through the width W of matrix layer 16 and the first low release silicon liners 19,20, the perforated matrix layer 16 with the perforated release liners 19,20, that expediently have conferred structural integrity to the matrix layer 16 at the perforation unit 21, proceeds to the third station 23 for delaminating the first low release silicon liner 19 that protects the adhesive 6 on the polyethylene layer 2's topside 4. The third station 23 comprises a first delaminating roll 24 for winding said first low release silicon liner 19 off the polyethylene layer 2 of the matrix layer 16, as indicated by the arrow C, and nip rollers 25 for advancing the partly delaminated matrix layer 16' through the production line 15.

The partly delaminated matrix layer 16' is transported to a fourth station 26, as indicated by the arrow D, in order to be laminated to a width of polyurethane laminate 27, which as indicated by the arrow E, is supplied by second roller means, the supply roll 28, to laminating rollers 29 to be laminated at a fifth station 30 with the delaminated matrix layer 16' having the first low release liner 20 on the hydrogel side 10. The polyurethane laminate 27 consists of a width of polyurethane foam layer 14a laminated to a width of breathable polyurethane film layer 14b, which polyurethane laminate 27 is supported on a paper backing 31 on the polyethylene film layer 14b side.

The delaminated matrix layer 16a' and the polyurethane laminate 27 together forms a width of wound care dressing material 32, intermediately protected at the hydrogel 7 side 10 by first low release liner 20 and on the polyurethane film 2 layer side by the paper backing 31.

As indicated by arrow F the wound care dressing material 32, which still are protected on both sides by either a release liner 20 or a paper backing 31, are stripped from said first low release silicon liner 20 and the paper backing 31 at a sixth station 33, a delamination/lamination station 33. The delamination part of the delamination/lamination station 33 consists of nip rollers 35 for advancing the wound dressing material 32 and delaminating rollers 36a,36b for winding the paper backing 31 and the first low release silicon liner 20, respectively, as indicated with respective arrows G and H, off the wound care dressing material 32.

Finally, at a lamination part of the delamination/lamination station 33 the second low release silicon liner 11 is applied to the skin-facing side 10 of the hydrogel 7 by conveying the delaminated wound dressing material 34 to a lamination unit 37. As indicated by arrows I and J, respectively, a minor low release silicon liner flap 13 is supplied by supply roll 38 and a major low release liner silicon flap is supplied by supply roll 39 to laminating rollers 40 for applying easy peelable release liners 12,13 to the hydrogel side 10 of the delaminated wound care dressing material 34.

Wound care dressings 1 are then continuously die cut with selected outlines from the re-laminated wound care dressing material 32' at a seventh station, an end cutting station 41.

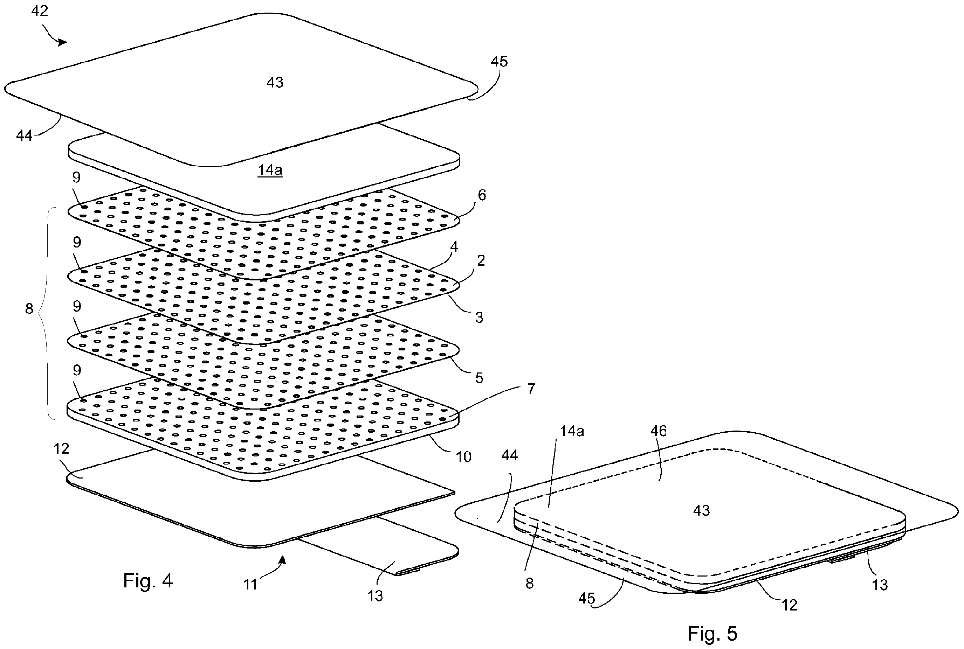

Fig. 4 illustrates a second embodiment of a wound care dressing 42 according to the present invention. The second embodiment 42 corresponds substantially to the first embodiment and differs only in that the wound care dressing is configured as an island pad dressing where the cover film layer sheet 43 extends with an adhesive border 44 beyond the perimeter of the rest of the laminated layer sheets, including the polyethylene layer sheet 2, the hydrogel layer sheet 7, the foam layer sheet 14a, and the cover layer film sheet 43. The adhesive border 44 is provided with an adhesive 45 to attach better to the skin surrounding the wound on which the wound care dressing 42 is applied for use.

Fig. 5 shows the same in assembled state illustrating the larger area of the cover film layer sheet 43 than the island pad 46, which is seen in broken line through said cover film layer sheet 43.

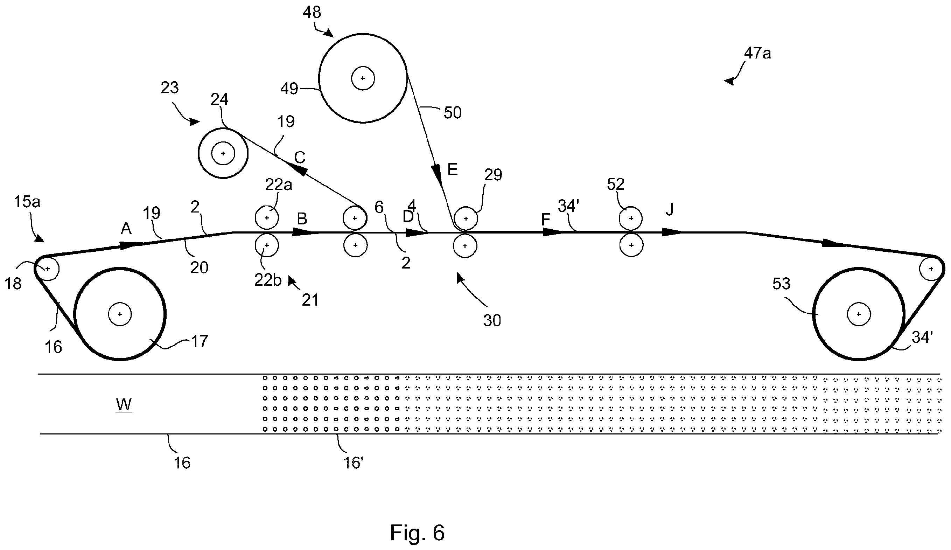

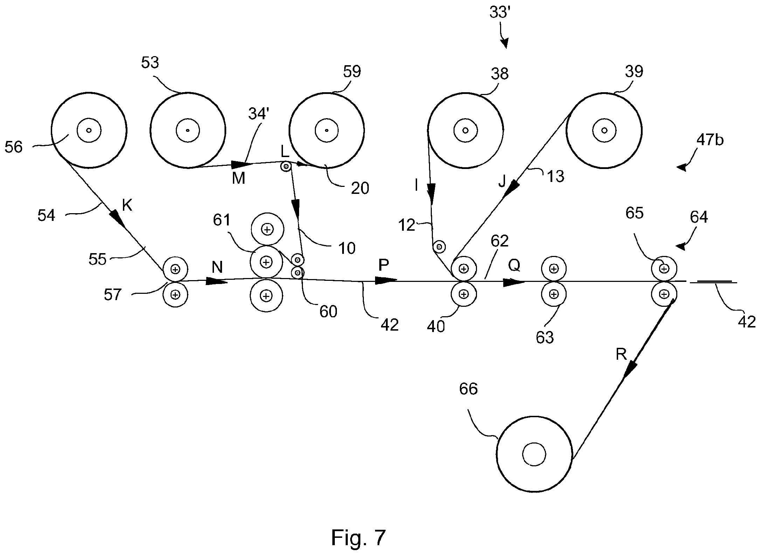

Figs. 6 and 7 shows schematically a production line 47 including two production subsections 47a,47b for the island pad embodiment 42 of a wound care dressing according to the present invention. The two production section 47a,47b includes substantially the same stations as the production line 15 described for manufacturing the first embodiment of a wound care dressing 1, and for like part same reference numerals are used.

In particular the first production section 47a of the production line 47a,47b includes the same first station 15a, second station 21, third station 23 and fifth station 39, and said stations are not discussed anew.

The first production section 47a has a fourth station 48 including a second roller means 49 for supplying the width of polyurethane foam 50, said width being unsupported by backing layers or release liners. Utilising the adhesive 6 on the topside 4 of the polyethylene film layer 2, the polyurethane foam layer 50 is laminated to the partly delaminated, perforated width of matrix layer. A further set of lamination and nip rollers 52 is provided for conveying the semifinished wound dressing material 34' to an end bobbin 53, as indicated by the arrow J.

The semifinished wound dressing material 34' is temporarily stored on the end bobbin 53 before said semifinished wound dressing material 34' is to be used in the second production section 47b of the production line 47, which second production section 47b is schematically shown in fig. 7.

A width of polyurethane film layer 54 on a low releasable paper backing layer 55 is supplied to the second section 47b of the production line 47a,47b from supply roll 56, as indicated by arrow K, and conveyed further into the production line by means of nip rollers 57 for being laminated to island pads prepared from the semifinished wound dressing material 34', as described below.

Said semifinished wound dressing material 34' is delivered from the end bobbin 53, as indicated by arrow M, the first low release liner 20 on the hydrogel side 10 of the semifinished wound dressing material 34' is delaminated, as indicated by arrow L, and wound on waste roller 59. The delaminated semifinished wound dressing material 34' is conveyed to diecutter 60 for producing islands pads 42. The cut out island pads 42 are conveyed further on by laminating rollers 61 for being placed on the width of polyurethane film layer 54, conveyed by nip rollers 57, as indicated by the arrow N. Thereafter the island pads 42 proceeds further into a modified sixth station 33', as indicated by arrow P, for laminating a second release liner 12,13, in the form of two partly overlapping release liner flaps 12,13 onto the exposed exterior hydrogel side 10, as described above for the production line 15.

As indicated by the arrow Q, the width of island pad wound dressing material 62 is conveyed by means of nip rollers 63 further on to the end cutting station 64, the seventh station, where island pads embodiments 42 are die cut by means of die cutter 65.

The backing layer 55 on the width of polyurethane film layer 54 is wound up on waste roller 66.

Both the first embodiment 1 and the island pad embodiments 42, can proceed directly for packaging, in which case wound dressings are successively placed on a first wrapping part for being sandwiched between a second wrapping part in a continuous process at a station (not shown) subsequent the end cutting station 64.

Said subsequent station may include welding means for welding the wrapping parts together along a perimeter zone larger than the perimeter of the wound dressing, and the continuous width of packaged wound dressing can be cut into individual packages by cutting means. Additionally, the packaged wound care dressing products may be sterilised if desired.

## Claims

1. A wound care dressing (1;42) comprising at least
- a separating film layer sheet (2) having a topside (4) and a bottom side (3) both provided with an adhesive (5,6),
- a hydrogel layer sheet (7) attached to the bottom side (3) of the separating film layer sheet (2) to form a combined matrix layer (16), and where
- the matrix layer (16) has a plurality of perforations (9),
**characterised in that**
- a foam layer sheet (14a) is a polyurethane foam layer sheet attached to the topside (4) of the separating layer sheet (2) and covered by a cover film layer sheet (14b;43), which cover film layer sheet (14b;43) is a polyurethane film layer sheet that allows passage of water vapor.

2. A wound care dressing (1;42) according to claim 1, **characterised in that** the foam layer sheet (14a) is, has a thickness of between 2 - 7 mm, preferably between 3 - 6 mm, and most preferred between 4 - 5 mm.

3. A wound care dressing (1;42) according to claim 1 or 2, **characterised in that** the polyurethane film layer sheet is bacteria proof and/or water proof.

4. A wound care dressing (1;42) according to any of the preceding claims 1 - 3, **characterised in that** the thickness of the cover film layer sheet (14b;43) is between 15 - 35 µm, preferably between 20 - 30 µm, and most preferred between 22 - 28 µm, and even more preferred 25 µm.

5. A wound care dressing (1;42) according to any of the preceding claims 1 - 4, **characterised in that** the separating film layer sheet (2) is a polyethylene film layer sheet or a polyurethane layer sheet wherein the thickness of the separating film layer sheet (2) including the adhesive (5,6) is between 40 - 100 µm, preferably between 50 - 80 µm, and most preferred between 60 - 70 µm.

6. A wound care dressing (1;42) according to any of the preceding claims 1 - 5, **characterised in that** the thickness of the hydrogel layer sheet (7) is 1 - 5 mm, preferably 2 - 4 mm, and most preferred between 2.5 - 3.5 mm, preferably the hydrogel layer sheet (7) is non-tacky

7. A wound care dressing (1;42) according to any of the preceding claims 1 - 6, **characterised in that** the foam of the foam layer sheet (14a) has an MVTR of at least 3500g/m²/24 hour.

8. A wound care dressing (1;42) according to any of the preceding claims 1 - 7, **characterised in that** the perforations (9) through the matrix layer (16) is made by ultrasonic perforation.

9. A wound care dressing (1;42) according to any of the preceding claims 1 - 8, **characterised in that** the perforations (9) are substantially equally distributed over the area of the matrix layer (16).

10. A wound care dressing (1;42) according to any of the preceding claims 1 - 9, **characterised in that** the perforations (9) have a diameter or transverse section of substantially between 2 - 4 mm, preferably between 2.5 - 3.5 mm and most preferred 3 mm.

11. A wound care dressing (1;42) according to any of the preceding claims 1 - 10, **characterised in that** the perforations (9) are provided at a mutual distance of about 8 - 10 mm.

12. A wound care dressing (1;42) according to any of the preceding claims 1 - 11, **characterised in that** the number of perforations (9) are about 80 - 120/ 100 cm² matrix layer (16).

13. A wound care dressing (1;42) according to any of the preceding claims 1 - 12, **characterised in that** the side (10) of the matrix layer (16) facing opposite the foam layer sheet (14a) is provided with a first release liner (11), optionally the release liner (11) comprises a first flap (12) and a second flap (13), optionally the first flap (12) and the second flap (13) overlap at least partly, optionally the first release liner is a low release silicon liner.

14. A wound care dressing (42) according to any of the preceding claims 1 - 13, **characterised in that** the cover layer film sheet (43) extends with a border (44) beyond the perimeter of an island pad (46) consisting of at least the combined matrix layer (16) and foam layer sheet (14a).

15. A wound care dressing (42) according to claim 14, **characterised in that** the border (44) is provided with adhesive (45).

## Patentansprüche

1. Wundverband (1;42), der mindestens umfasst:
eine trennende Filmschichtlage (2) mit einer Oberseite (4) und einer Unterseite (3), die beide mit einem Haftmittel (5,6) versehen sind,
eine Hydrogel-Schichtlage (7), die an der Unterseite (3) der trennenden Filmschichtlage (2) befestigt ist, um eine kombinierte Matrixschicht (16) zu bilden, und wobei
die Matrixschicht (16) mehrere Perforationen (9) aufweist,
**dadurch gekennzeichnet, dass**
eine Schaumschichtlage (14a) eine Polyurethanschaum-Schichtlage ist, die an der Oberseite (4) der trennenden Schichtlage (2) befestigt ist und von einer Deckfilmschichtlage (14b;43) bedeckt ist, wobei die Deckfilmschichtlage (14b;43) eine Polyurethanfilm-Schichtlage ist, die einen Durchlass von Wasserdampf ermöglicht.

2. Wundverband (1;42) nach Anspruch 1, **dadurch gekennzeichnet ist, dass** die Schaumschichtlage (14a) eine Dicke zwischen 2 bis 7 mm aufweist, vorzugsweise zwischen 3 bis 6 mm, und am meisten bevorzugt zwischen 4 bis 5 mm.

3. Wundverband (1;42) nach Anspruch 1 oder 2, **dadurch gekennzeichnet, dass** die Polyurethanfilm-Schichtlage bakteriendicht und/oder wasserdicht ist.

4. Wundverband (1;42) nach einem der vorhergehenden Ansprüche 1 bis 3, **dadurch gekennzeichnet, dass** die Dicke der Deckfilmschichtlage (14b;43) zwischen 15 bis 35 µm liegt, vorzugsweise zwischen 20 bis 30 µm, und am meisten bevorzugt zwischen 22 bis 28 µm, und noch mehr bevorzugt 25 µm.

5. Wundverband (1;42) nach einem der vorhergehenden Ansprüche 1 bis 4, **dadurch gekennzeichnet, dass** die trennende Filmschichtlage (2) eine Polyethylenfilm-Schichtlage oder eine Polyurethan-Schichtlage ist, wobei die Dicke der trennenden Filmschichtlage (2) einschließlich des Haftmittels (5,6) zwischen 40 bis 100 µm liegt, vorzugsweise zwischen 50 bis 80 µm, und am meisten bevorzugt zwischen 60 bis 70 µm.

6. Wundverband (1;42) nach einem der vorhergehenden Ansprüche 1 bis 5, **dadurch gekennzeichnet, dass** die Dicke der Hydrogel-Schichtlage (7) bei 1 bis 5 mm liegt, vorzugsweise bei 2 bis 4 mm, und am meisten bevorzugt zwischen 2,5 bis 3,5 mm, wobei die Hydrogel-Schichtlage (7) vorzugsweise nicht klebrig ist.

7. Wundverband (1;42) nach einem der vorhergehenden Ansprüche 1 bis 6, **dadurch gekennzeichnet, dass** der Schaum der Schaumschichtlage (14a) eine MVTR (Wasserdampfdurchlässigkeit) von mindestens 3.500 g/m²/24 Stunden aufweist.

8. Wundverband (1;42) nach einem der vorhergehenden Ansprüche 1 bis 7, **dadurch gekennzeichnet, dass** die Perforationen (9) durch die Matrixschicht (16) durch eine Ultraschallperforation hergestellt sind.

9. Wundverband (1;42) nach einem der vorhergehenden Ansprüche 1 bis 8, **dadurch gekennzeichnet, dass** die Perforationen (9) im Wesentlichen gleichmäßig über die Fläche der Matrixschicht (16) verteilt sind.

10. Wundverband (1;42) nach einem der vorhergehenden Ansprüche 1 bis 9, **dadurch gekennzeichnet, dass** die Perforationen (9) einen Durchmesser oder einen Querschnittsabschnitt von im Wesentlichen zwischen 2 bis 4 mm, vorzugsweise zwischen 2,5 bis 3,5 mm und am meisten bevorzugt 3 mm aufweisen.

11. Wundverband (1;42) nach einem der vorhergehenden Ansprüche 1 bis 10, **dadurch gekennzeichnet, dass** die Perforationen (9) in einem gegenseitigen Abstand von etwa 8 bis 10 mm bereitgestellt werden.

12. Wundverband (1;42) nach einem der vorhergehenden Ansprüche 1 bis 11, **dadurch gekennzeichnet, dass** die Anzahl der Perforationen (9) bei etwa 80 bis 120/100 cm² Matrixschicht (16) liegt.

13. Wundverband (1;42) nach einem der vorhergehenden Ansprüche 1 bis 12, **dadurch gekennzeichnet, dass** die Seite (10) der Matrixschicht (16), die gegenüber der Schaumschichtlage (14a) liegt, mit einem ersten Ablöseüberzug (11) versehen ist, wobei der Ablöseüberzug (11) wahlweise eine erste Lasche (12) und eine zweite Lasche (13) umfasst, wobei wahlweise die erste Lasche (12) und die zweite Lasche (13) mindestens teilweise überlappen, wobei der erste Ablöseüberzug ein geringer Siliciumablöseüberzug ist.

14. Wundverband (42) nach einem der vorhergehenden Ansprüche 1 bis 13, **dadurch gekennzeichnet, dass** sich die Deckschichtfilmlage (43) mit einem Rand (44) über die Umfangslänge eines Inselpolsters (46) erstreckt, das aus mindestens der kombinierten Matrixschicht (16) und der Schaumschichtlage (14a) besteht.

15. Wundverband (42) nach Anspruch 14, **dadurch gekennzeichnet, dass** der Rand (44) mit einem Haftmittel (45) versehen.

## Revendications

1. Pansement pour le traitement de plaies (1 ; 42) comprenant au moins :
- une feuille faisant office de couche de séparation (2) sous la forme d'un film possédant un côté supérieur (4) et un côté inférieur (3), tous deux munis d'un adhésif (5, 6) ;
- une feuille faisant office de couche (7) sous la forme d'un hydrogel fixée au côté inférieur (3) de la feuille faisant office de couche de séparation (2) sous la forme d'un film dans le but d'obtenir une couche combinée (16) faisant office de matrice ; et dans lequel
- la couche (16) faisant office de matrice possède plusieurs perforations (9) ;
**caractérisé en ce que**
- une feuille faisant office de couche (14a) sous la forme d'une mousse représente une feuille faisant office de couche sous la forme d'une mousse de polyuréthane fixée au côté supérieur (4) de la feuille faisant office de couche de séparation (2) et recouverte d'une feuille faisant office de couche de recouvrement (14b ; 43) sous la forme d'un film, ladite feuille faisant office de couche de recouvrement (14b; 43) sous la forme d'un film représentant une feuille faisant office de couche sous la forme d'un film en polyuréthane qui permet le passage de la vapeur d'eau.

2. Pansement pour le traitement de plaies (1 ; 42) selon la revendication 1, **caractérisé en ce que** la feuille faisant office de couche (14a) sous la forme d'une mousse possède une épaisseur entre 2 et 7 mm, de préférence entre 3 et 6 mm et de manière de loin préférée entre 4 et 5 mm.

3. Pansement pour le traitement de plaies (1 ; 42) selon la revendication 1 ou 2, **caractérisé en ce que** la feuille faisant office de couche sous la forme d'un film de polyuréthane possède une étanchéité aux bactéries et/ou une étanchéité à l'eau.

4. Pansement pour le traitement de plaies (1 ; 42) selon l'une quelconque des revendications précédentes 1 à 3, **caractérisé en ce que** l'épaisseur de la feuille faisant office de couche de recouvrement (14b; 43) sous la forme d'un film se situe entre 15 et 35 µm, de préférence entre 20 et 30 µm et de manière de loin préférée entre 22 et 28 µm, et de manière encore plus préférée s'élève à 25 µm.

5. Pansement pour le traitement de plaies (1 ; 42) selon l'une quelconque des revendications précédentes 1 à 4, **caractérisé en ce que** la feuille faisant office de couche de séparation (2) sous la forme d'un film représente une feuille faisant office de couche sous la forme d'un film de polyéthylène ou une feuille faisant office de couche de polyuréthane ; dans lequel l'épaisseur de la feuille faisant office de couche de séparation (2) sous la forme d'un film, y compris les adhésifs (5, 6) se situe entre 40 et 100 µm, de préférence entre 50 et 80 µm et de manière de loin préférée entre 60 et 70 µm.

6. Pansement pour le traitement de plaies (1 ; 42) selon l'une quelconque des revendications précédentes 1 à 5, **caractérisé en ce que** l'épaisseur de la feuille faisant office de couche (7) sous la forme d'un hydrogel s'élève de 1 à 5 mm, de préférence de 2 à 4 mm, et de manière de loin préférée se situe entre 2,5 et 3,5 mm ; de préférence, la feuille faisant office de couche (7) sous la forme d'un hydrogel est non collante.

7. Pansement pour le traitement de plaies (1 ; 42) selon l'une quelconque des revendications précédentes 1 à 6, **caractérisé en ce que** la mousse de la feuille faisant office de couche (14a) sous la forme d'une mousse possède une valeur MVTR s'élevant à au moins 3500 g/m²/24 heures.

8. Pansement pour le traitement de plaies (1 ; 42) selon l'une quelconque des revendications précédentes 1 à 7, **caractérisé en ce que** les perforations (9) à travers la couche (16) faisant office de matrice sont réalisées par l'intermédiaire d'une perforation par ultrasons.

9. Pansement pour le traitement de plaies (1 ; 42) selon l'une quelconque des revendications précédentes 1 à 8, **caractérisé en ce que** les perforations (9) sont distribuées de manière essentiellement égale sur la surface de la couche (16) faisant office de matrice.

10. Pansement pour le traitement de plaies (1 ; 42) selon l'une quelconque des revendications précédentes 1 à 9, **caractérisé en ce que** les perforations (9) possèdent un diamètre ou une section transversale qui se situe essentiellement entre 2 et 4 mm, de préférence entre 2,5 et 3,5 mm et qui s'élève de manière de loin préférée à 3 mm.

11. Pansement pour le traitement de plaies (1 ; 42) selon l'une quelconque des revendications précédentes 1 à 10, **caractérisé en ce que** les perforations (9) sont prévues à une distance réciproque qui s'élève d'environ 8 à 10 mm.

12. Pansement pour le traitement de plaies (1 ; 42) selon l'une quelconque des revendications précédentes 1 à 11, **caractérisé en ce que** le nombre des perforations (9) s'élève d'environ 80 à 120/100 cm² de la couche (16) faisant office de matrice.

13. Pansement pour le traitement de plaies (1 ; 42) selon l'une quelconque des revendications précédentes 1 à 12, **caractérisé en ce que** le côté (10) de la couche (16) faisant office de matrice, orientée à l'opposé de la feuille faisant office de couche (14a) sous la forme d'une mousse est muni d'un premier revêtement antiadhésif (11); de manière facultative, le revêtement antiadhésif (11) comprend un premier rabat (12) et un deuxième rabat (13); de manière facultative, le premier rabat (12) et le deuxième rabat (13) se chevauchent au moins en partie ; de manière facultative, le premier revêtement antiadhésif représente un revêtement à base de silicone qui se décolle lentement.

14. Pansement pour le traitement de plaies (42) selon l'une quelconque des revendications précédentes 1 à 13, **caractérisé en ce que** la feuille faisant office de couche de recouvrement (43) sous la forme d'un film s'étend avec un bord (44) qui se situe au-delà du périmètre d'une compresse isolante (46) constituée par au moins la couche combinée (16) faisant office de matrice et par la feuille faisant office de couche (14a) sous la forme d'une mousse.

15. Pansement pour le traitement de plaies (42) selon la revendication 14, **caractérisé en ce que** le bord (44) est muni d'un adhésif (45).
